(19) Europäisches Patentamt
European Patent Office
Office européen des brevets

(11) Publication number: **0 175 490 A2**

(12) **EUROPEAN PATENT APPLICATION**

(21) Application number: **85305918.6**

(22) Date of filing: **20.08.85**

(51) Int. Cl.⁴: **A 61 L 2/18**, A 01 N 43/54 // (A01N43/54, 31:04)

(30) Priority: **18.10.84 US 662053**
**20.08.84 US 641996**
**20.08.84 US 642088**

(43) Date of publication of application: **26.03.86**
**Bulletin 86/13**

(84) Designated Contracting States: **AT BE CH DE FR GB IT LI LU NL SE**

(71) Applicant: **SHERMAN LABORATORIES, INC., P.O. Box 368, Abita Springs Louisiana 70420 (US)**

(72) Inventor: **Sherman, Guy J., 3015 Highway 59 Mandeville, St. Tammany Louisiana (US)**

(74) Representative: **Perry, Robert Edward et al, GILL JENNINGS & EVERY 53-64 Chancery Lane, London WC2A 1HN (GB)**

(54) **Contact lens treatment compositions and their uses.**

(57) Trimethoprim is used as an active ingredient in sterile aqueous compositions for disinfecting, wetting and/or cleaning contact lenses, especially soft contact lenses. For disinfection and wetting at least, benzyl alcohol and, optionally, EDTA or a salt thereof are used in addition to trimethoprim.

EP 0 175 490 A2

# CONTACT LENS TREATMENT COMPOSITIONS AND THEIR USE

This invention relates to compositions for treating contact lens, and to such treatment.

Just as there are marked differences in the structure and composition of hard PMMA, silicone, silicone copolymer gas-permeable contact lenses and soft contact lenses, there are also marked differences in the maintenance, care and treatment of the various types of hard, silicone, silicone copolymer and soft lenses. While patient care and treatment of hard contact or conventional contact lenses is relatively simple and uncomplicated, the proper care and treatment of silicone and silicone copolymer lenses (gas-permeable) and the newer soft and hydrophilic lenses have proved to be more complex, time-consuming and costly to the patient.

The primary difference between the conventional hard contact lenses and the silicone copolymer lenses and more complex soft lenses is the hydrophilic nature of the silicone copolymer lenses, and the markedly higher number of polar or water-attracting centres of the hydrophilic gel material from which the soft contact lenses are made. Soft lenses, as a result, have unique physical properties and clinical behaviour. This polar or water-attracting centre of the gel material is represented in the hydroxyethyl methacrylate bond as a hydroxyl group (-OH) which attracts and holds large amounts of water. It is this high water content, held in the expanded matrix of the hydrophilic gel lens, which leads to especial difficulties in cleaning and disinfecting or asepticising the soft hydrophilic lens.

The hydrophilic nature of soft contact lenses makes them vulnerable to bacterial contamination. While studies have demonstrated that bacteria cannot penetrate the intramolecular pores of the hydrophilic lens, except in defective lenses, the bacteria have an affinity for

protein and tear deposits on the surfaces of the lens matrix. In particular, the tears and fluids absorbed by the soft lenses serve as a bacterial culture medium. If defects or nicks occur in the lenses during either manufacture or subsequent patient wear, bacteria may find a haven to grow and be sheltered from superficial lens cleaning and disinfection.

Potentially harmful fungi are also a possible danger to the soft contact lens. Fungi, like bacteria, can thrive in tear secretions, other fluids or deposits. They can penetrate the lens material directly if enzymatic degradation of the lens material has taken place. Similarly, any substantial residual proteinaceous or tear secretion deposits or lipid deposits remaining in or on the lens may readily overwhelm and inactivate the most effective germicidal components of a disinfecting system.

Problems can accrue from incorrect and careless handling of soft lenses by the user. Many potential contaminants and lens deposits can be transferred from unwashed fingers to the surface of a soft lens. Such contaminants include oily deposits from the skin, sweat, skin lotions and creams, mascara, detergents, lipstick and even nicotine. Controlled studies have demonstrated that bacterial contaminants occur in 43% of the make-up used by women, and fungal contaminants in 12%. Attempts to effect sterilization of the lenses by boiling, for example, can be cumbersome, in addition to causing permanent damage to the lenses if done improperly. If the patient has used impure water for storage and rinsing of the lenses, undesirable deposits such as calcium, iron and insoluble divalent and trivalent metal salts as well as other chemical deposits can collect on the lens surfaces.

It is therefore important that, prior to storing soft contact lenses in a disinfecting solution, protein and lipid deposits are removed from the lens surfaces. Effective cleaning is thus an essential part of any effective soft lens treatment and maintenance regimen.

Numerous methods of cleaning soft contact lenses remove only protein deposits. For example, the soft lenses can be rinsed in tap water, in an attempt to remove protein deposits, but only 1 to 10% of the accumulated debris on the lens surfaces is removed. Boiling the soft contact lenses in a saline solution is partially effective, generally removing from 5 to 15% of the debris.

Other cleaning methods are known. Hydrogen peroxide is unsatisfactory, since it can oxidise and change the colour of the lenses. The use of high concentrations of sodium chloride removes some encrustations, owing to the friction created in rubbing, but scratching of the lens surfaces occurs. Enzymatic cleaners do not remove deposits such as salts, lipids and mucin, and may also discolour the soft contact lenses. A more detailed discussion of various types of cleaning procedures and compositions is found in Review of Optometry (April 1978) in respective articles by Arons, Lieblein, and Kleist and Thorson.

Wetting solutions are commonly used to prepare contact lenses prior to insertion into the eye. Known contact lens wetting solutions comprise polyvinyl alcohol as a wetting agent, methylcellulose or hydroxyethylcellulose as a viscosity-building agent, and also a sufficient amount of one or more water-soluble salts, generally sodium chloride, to make them isotonic or hypertonic with respect to human serum and tear fluid. For example, hypertonic wetting solutions are disclosed in US-A-3549747.

Re-wetting solutions are applied directly into the eyes when contact lenses are being worn. Such solutions can also be used before or after wearing periods. Re-wetting provides comfort and re-lubrication for the eye.

Since contact lenses are susceptible to bacteria and fungi, a cleaner or disinfectant solution should contain a preservative against contamination. Wetting and re-wetting solutions generally include a preservative system, to prevent or inhibit microbial growth, especially where multi-dose containers of the solution are prepared. Generally, where no preservative is employed, single dose containers are utilized, which results in greater expense.

US-A-3549747 discloses a known preservative system for wetting solutions. Various antiseptic agents provide rapid and effective kill of a broad range of micro-organisms, but have proved to be unsuitable for use in soft lens treatment solutions; these agents are incompatible with the soft lens material, bind with protein deposits on the surface of the lens matrix, or are concentrated by the lens material to the extent that they cause discomfort and potential damage to the corneal surface of the wearer's eyes. For example, benzalkonium chloride is unacceptable because it binds with many types of soft lens material and binds with protein deposits on the lens surface.

One type of cold disinfecting solution for soft contact lenses uses chlorohexidine. However, chlorohexidine is absorbed by the soft contact lens material. It gradually passes into the eye, often causing excessive burning, irritation and red eye, in addition to discoloring soft lenses.

Further, compounds such as thimerosal, potassium sorbate, and sorbic acid have been used as preservatives.

However, these compounds have drawbacks, in that they can discolour lenses; they can also concentrate in the lens matrix and cause irritation, sensitisation, excessive burning and red eye. With the advent of extended wear lenses, it becomes especially important to avoid such problems.

A sterile contact lens cleaning composition, according to the present invention, comprises trimethoprim and a cleaning component.

A sterile contact lens disinfectant composition, according to another aspect of the invention, comprises trimethoprim and benzyl alcohol.

A sterile contact lens wetting composition, according to a further aspect of the invention, comprises trimethoprim, benzyl alcohol and a wetting agent.

A cleaning composition of the invention includes a preservative system which avoids hypersensitivity problems associated with preservative systems containing, for example, sorbic acid, potassium sorbate, thimerosal or chlorhexidine. Novel compositions are suitable for hard contact lenses, and especially suitable for use on silicone, silicone copolymer and soft lenses (such as HEMA contact lenses) including extended wear contact lenses, without causing discoloration or being deleterious to soft tissue, e.g. as the result of lens absorption.

Novel wetting compositions can be used by those who do not wear contact lenses, for the relief of, say, dry eye syndromes and idiopathic ocular discomfort. The terms "wetting solution" and "wetting composition" as used herein include re-wetting solutions or compositions for application directly into the eye.

A cleaning composition of the invention can be used to remove proteins, lipids and other foreign deposits from the surfaces of a contact lens, to eliminate

interference with wearing comfort, and to maintain clear vision. The cleaning composition is simple to use and can be easily rinsed from a contact lens after the cleaning is completed.

In a contact lens cleaning composition of the invention, various adjuvant bactericides may be present, but it has been discovered that trimethoprim alone is effective in maintaining the sterility of a contact lens cleaning composition. Adjuvant bactericides that can optionally be included are ethylenediaminetetraacetic acid (EDTA), or a water-soluble salt thereof, and sorbic acid. Ascorbic acid, or a salt thereof, and/or benzyl alcohol, may be utilised in place of or in addition to sorbic acid. Trimethoprim, benzyl alcohol and, optionally, EDTA are satisfactory for a wetting or disinfectant composition; "disinfectant" means that bacteria, fungi, yeasts and viruses are killed.

The use of appropriate antiseptic, bactericidal and fungicidal chemical agents requires that the selected chemical agents be compatible with all other components of the solution, as well as with the contact lens material. The essential considerations in determining the optimum anti-bacterial and anti-fungal agents are that they should not bind to protein, and should not react with or be absorbed in the soft lens material or matrix. The preservative systems used in the invention meet these requirements.

An effective amount of trimethoprim for a cleaning composition of the invention is from 0.05 to 2.0%, more preferably 0.075 to 0.3%, and most preferably about 1%, by weight of the total composition. For a wetting composition, the amount of trimethoprim is usually 0.01 to 2.0%, and preferably about 0.025%, by weight of the composition. For disinfectant use, the trimethoprim concentration may be from 0.01 to 4.0% by weight.

In cleaning and wetting compositions of the invention, materials which can act as adjuvant bactericides are generally present as follows: EDTA, from 0.025 to 0.5%; sorbic acid (for cleaning compositions), from 0.001 to 0.35%; benzyl alcohol, from 0.1 to 5.0%, all by weight of the total composition. For sterility, higher concentrations of these compounds can be used. Benzyl alcohol aids dissolution of trimethoprim in the composition.

The inclusion of EDTA, or a water-soluble salt thereof, serves as a buffering and preservative component of a cleaning, wetting or bactericidal composition used according to the invention, and provides anti-bacterial and anti-fungal properties. The preferred EDTA salt is the disodium salt (disodium EDTA or disodium edetate). Other salts of EDTA which may be used include, for example, mono-, di-, tri- and tetra-alkali metal salts.

In a cleaning composition of the invention, ascorbic acid, or a salt thereof, may be present, usually in an amount of from 0.1 to 20%, calculated as ascorbic acid. Suitable salts of ascorbic acid include the sodium and calcium salts.

When ascorbic acid is used, monothioglycerol is preferably included, in an amount effective to stabilise the ascorbic acid compound. Most preferably, in this embodiment, the weight ratio of monothioglycerol to the ascorbic acid compound, calculated on the basis of ascorbic acid, is 1:50.

When ascorbic acid is used in order to increase the shelf-life, the compositions are formulated and packaged in an atmosphere which is substantially devoid of free oxygen. For example, the compositions can be formulated

and sealed in sterile containers, in the presence of a nitrogen or carbon dioxide atmosphere. Further, it is advantageous for the ascorbic acid compound to be packaged in a non-transparent container, to reduce degradation which can be caused by ultraviolet radiation. Ascorbic acid could also be packaged separately, until the time of use.

The preferred prophylactic cleaning and disinfectant compositions of the present invention also include propylene glycol, e.g. in an amount of from 0.005 to 5.0%, and preferably 0.2 to 2.5%, e.g. about 1%, by weight of the total aqueous composition. Propylene glycol acts as a humectant, increases the viscosity, body and feel of the cleaner, without interfering with the detergent action of a detergent cleaning system. Propylene glycol also acts as a preservative and fungal growth inhibitor, and reduces fogging of soft contact lenses. Another important function of propylene glycol in a prophylactic cleaning composition of the invention is that it permits the composition to be easily rinsed from the lenses after cleaning.

Any suitable detergent or other type of cleaning component may be used, which is suitable for cleaning contact lenses, e.g. for soft contact lenses. A non-ionic (preferred), anionic or cationic detergent material may be used.

The surfactants which are employed are generally completely miscible with water at the concentrations employed, and generally provide a clear solution. In addition, the surfactant must be stable under the disinfecting conditions, should act adversely with neither the contact lens nor other materials present in the solution and, finally, must not irritate the eye. Therefore, the surfactant must not be adsorbed by a soft contact lens, while preferably being capable of

solubilising the proteinaceous and lipid materials adsorbed on the lens, and preventing redeposition during the disinfection treatment and subsequent storage.

One group of suitable surfactants is the non-ionic surfactants, particularly hydroxyalkylated and polyoxyalkylated surfactants. For effectiveness at very low concentrations, N-hydroxyalkylated carboxamides of $C_{10-18}$, preferably $C_{12-14}$, fatty acids are suitable; they are preferably saturated, but may contain one olefinic unsaturation site. There will normally be two $C_{2-3}$ hydroxyalkyl groups which may be the same or different.

Suitable polyoxyalkylated non-ionic detergents are solely poly($C_{2-3}$ oxyalkylene) groups or may have a polyoxyalkylene chain bonded directly or indirectly to a $C_{10-18}$ aliphatic chain. The alkyl-containing group may be, for example, a sorbitan ester, alkylphenyl, alkyl or carboxylic acid group. The polyoxyalkylene chain may be a homo-oligomer or co-oligomer, with the homo-oligomer normally being of ethyleneoxy groups and the co-oligomer being a random or block co-oligomer of ethyleneoxy and propyleneoxy groups. These various non-ionic detergents are widely commercially-available. The alkyleneoxy chains will generally have, on average, 5 to 60 oxyalkylene units.

Suitable ampholytic detergents are betaines having a $C_{10-18}$, preferably $C_{10-14}$, chain bonded to the nitrogen atom. Compounds of the formula

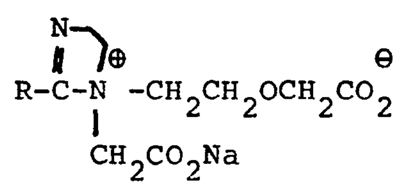

wherein R has from 9 to 13,, and especially 11, carbon atoms are preferred.

More specifically, the non-ionic cleaner may be a polyoxypropylene-polyoxyethylene block copolymer. Suitable block copolymers include those sold under the

trade name "Pluronic" by the BASF-Wyandotte Chemical Corp., USA, particular examples being the Pluronics F-68, F-77, P-75, P-65, L-64, F-87, F-88, F-98, F-108 and F-127. Examples of suitable amphoteric surfactants and methods of making them are found in US-A-2781349, US-A-2781350, US-A-2781351, US-A-3231580, US-A-3231581, US-A-3452042, US-A-3658895 and US-A-3697452. Especially suitable amphoteric surfactants are available from the Miranol Chemical Company, Inc., USA, under the trade name Miranol Amphoteric Surface Active Agents. One specific amphoteric surfactant is available as Miranol $H_2M$ concentrate.

Preferably, a non-ionic detergent system for use in a cleaning composition of the invention comprises three distinct types of non-ionic detergents. One type is polyoxypropylene-polyoxyethylene block copolymer, e.g. having a molecular weight of 1,100 to 14,000 and a water-solubility of more than 100 g/l. Preferably, the polyoxyethylene or hydrophilic unit of the block copolymer constitutes 70 to 85%, e.g. 70 to 80%, of the total molecular weight of the block copolymer, the remainder of the block copolymer being composed of polyoxypropylene (or another hydrophobic unit). Such a block copolymer is generally present in an amount of from 1.0 to 15.0%, preferably 6%, by weight of the total aqueous composition. Preferably, the block copolymer has relatively low foaming characteristics.

The second type of surfactant in the preferred non-ionic detergent system is an amphoteric surface active agent, preferably 2-cocoyl-2-imidazolinium lauryl sulfate-1-carboxymethyloxyethyl-1-carboxymethyl disodium which is also sold under the trade name "Miranol 2 MCA Modified" by the Miranol Chemical Company, Inc. of Irvington, New Jersey, USA. "Miranol MHT" is an alternative. The amount of the amphoteric surface active

agent is preferably from 0.5 to 8.0%, e.g. about 3.0%, by weight of the total aqueous composition.

The third type of detergent in the system is an alkylaryl polyether alcohol which should be water-soluble. It is preferably an octylphenolethylene oxide or isooctylphenoxypolyethoxyethanol which preferably contains about 9 units of ethoxyethanol per unit of isooctylphenol and has a molecular weight of about 630. An exemplary alkylaryl polyether alcohol is sold under the trade name "Triton X-100" by the Rohm & Haas Company of Philadelphia, Pennsylvania, USA. The amount of the alkylaryl polyether alcohol is preferably from 0.005 to 5.0%, more preferably about 1.0%, by weight of the total aqueous composition. The alkylaryl polyether alcohol complements the cleansing characteristics of the block copolymer, and facilitates removal of ocular secretions, proteinaceous deposits and other materials which may be deposited upon the surfaces of a contact lens.

Instead of all three, the second and third types may be used together satisfactorily. Combinations of two or three surfactants can act synergically.

A wetting composition of the invention includes a preservative system and a wetting system. Any wetting component may be used, which is suitable for wetting contact lenses and, especially, soft contact lenses. The wetting system will generally be completely miscible with water at the concentrations used, to provide a clear solution. In addition, the wetting system must not act adversely with the type of contact lens with which use is intended, nor with other materials present in the solution. It must not irritate the eye.

The wetting system usually includes a viscosity-building agent and a wetting agent suitable for soft contact lenses. Many such agents are known.

Suitable viscosity-building agents include water-soluble cellulosic polymers which may be synthetic or natural. Such materials also assist in wetting the lenses. Suitable wetting agents include polyvinyl alcohol and polyvinylpyrrolidone.

Suitable cellulosic polymers include hydroxyethylcellulose, methylcellulose, carboxymethylcellulose and natural gums. Usually, the amount of cellulosic polymer is from 0.05 to 0.80% by weight of the composition.

Usually, the wetting composition will have a viscosity of 26 to 40 cps at 25°C. Medium grade cellulosic polymers are useful for achieving the desired viscosity.

Preferably, polyvinyl alcohol which is used is fully hydrolysed. Generally, polyvinyl alcohol is present in an amount of from 0.5 to 2.5% by weight of the total composition.

Preferably, an additional wetting compound, a polyvinylpyrrolidone polymer, is used, usually in an amount of from 0.5 to 2.0% by weight of the composition. A preferred hydroxethylcellulose is available from Hercules, Inc. of Wilmington, Delaware, USA, under the trade designation "250 H". A preferred polyvinylpyrrolidone is available from GAF Corporation of New York, New York, USA, under the name Plasdone C. A preferred polyvinyl alcohol is available from the Monsanto Company of St. Louis, Missouri, USA, under the name of "Galvatol"; this product is partially hydrolysed.

Wetting compositions of the invention, containing polyvinyl alcohol, polyvinylpyrrolidone and/or a cellulosic polymer of the type described, are especially useful for wetting silicone copolymer contact lenses after the lenses have been cleaned with a non-ionic detergent cleaner, e.g. a cleaner as described above or as disclosed in US-A-4510065.

It has been discovered that the surface or static electricity charge which may be present on silicone copolymer lenses is eliminated or neutralized by such non-ionic detergents, and makes the lenses wettable by the wetting compositions. Failure to eliminate or neutralize such charges can prevent such lenses from being adequately wetted.

Thus, in accordance with another aspect of the present invention, a method of wetting a silicone copolymer contact lens having a surface charge comprises neutralizing or eliminating the surface charge, and contacting the lens with a wetting composition containing at least one wetting component selected from polyvinyl alcohol, polyvinylpyrrolidone and cellulosic polymers. Suitable wetting components are as previously described herein. One procedure by which the surface charge can be neutralized or eliminated is by contact with the non-ionic detergent material which is then rinsed from the lens prior to wetting. Suitable non-ionic detergents include the hydroxyalkylated and polyoxyalkylated surfactants as described above and in US-A-4510065.

A cleaning or wetting composition of the invention preferably includes combinations of essentially neutral and alkaline salts compatible with ocular tissue and soft contact lens material which are water-soluble, preferably present in a concentration to provide an aqueous composition salt content equivalent to a desired tonicity. A tonicity of 1.0 to 2.0 is generally satisfactory. 1.2 to 1.7 is the preferred range for a cleaning composition and 1.0 to 1.45 for a wetting composition.

A disinfectant composition of the invention preferably includes an essentially neutral water-soluble compatible salt to provide tonicity equivalent to between 0.8% and 1.8% sodium chloride by weight of the total

aqueous composition, i.e. a tonicity which is about the same as or slightly higher than the tonicity of normal human tear fluid. Preferably, sodium chloride and potassium chloride are utilized, in a weight ratio of from 2:1 to 7:3, respectively.

A solution used in the present invention is usually mildly hypertonic, and this helps in the prevention of possible absorption into the lens matrix of foreign matter, protein, lipids and bacteria which could build up and cause contamination problems and deterioration and discoloration of the lens itself. Sodium chloride can be present in an amount from 0.05 to 2.0%, preferably (for a cleaning composition) about 0.748%, by weight of the total aqueous composition. Potassium chloride is preferably used in conjunction with sodium chloride and should generally be present in an amount of from 0.05 to about 2.0%, preferably (for a cleaning composition) about 0.28%, by weight of the total aqueous composition.

The major or remaining component of a cleaning or wetting composition of the invention is purified water. Cleaning compositions of the invention generally have a pH of from 6.0 to 7.0, and preferably from 6.0 to 6.5. This slightly alkaline pH helps to dissolve protein, and facilitates rinsing the composition from the lens. Wetting compositions of the invention are preferably buffered and are neutral or slightly acid, e.g. having a pH of from 7.0 to 8.0. For the adjustment of pH, sodium bicarbonate may be present, generally in an amount of from 0.01 to 3.0%, e.g. about 0.1%, by weight of a cleaning composition, or 0.05 to 3.0% by weight of a wetting solution. A buffer system may comprise sodium bicarbonate, sodium phosphate (tribasic) and sodium biphosphate.

In accordance with one embodiment of the present invention, a treatment method is provided for killing

bacteria associated with contact lenses. A contact lens is treated by storing the lens in an aqueous solution containing trimethoprim and benzyl alcohol, present together in an effective concentration for killing bacteria, for a period of time sufficient for the composition to kill at least a portion of the bacteria associated with the contact lens. For example, the lens is introduced into a container having a suitable disinfectant solution, generally after proper cleaning procedures. The lens is immersed. Generally, storing the lens for about six hours will provide sufficient disinfection of the contact lens or bacteria for wearing the lens. Thus, the method is ideal for overnight storage of contact lenses or storage between wearing periods. Less than six hours of storage may also be sufficient.

Heating of the solution or lens is not required. Thus, the solutions and method of the present invention allow a lens to be disinfected at ambient temperature, eliminating the possibility of deleterious effects which may occur when the lens is heated.

The compositions of the present invention are preferably utilised as part of a total patient regimen for maintaining and treating soft and semi-hard contact lenses. An effective method of contact lens storage is an important part of any effective soft or semi-hard contact lens treatment and maintenance regimen. Separate cleaning and disinfection of the lenses, e.g. using a cleaning composition of the present invention, helps to ensure that gross organic or inorganic deposits and pollutants, including proteins and lipids, are removed from the surfaces of the lenses, to minimise wearing discomfort and to maximise clear vision with soft contact lenses. The preferred cleaning compositions of the invention provide rapid and efficient cleaning of contact

lenses, without any need for excessive rubbing, and easy rinsing after cleaning. Wear and tear are thus minimised. The need for an enzymatic cleaner is obviated.

By way of example of a rinsing and cold disinfecting solution for use in the invention, the following were formulated (amounts in % w/w):

| | |
|---|---|
| Sodium bicarbonate | 0.100 |
| Sodium phosphate (tribasic) | 0.030 |
| Sodium biphosphate | 0.030 |
| Sodium chloride | 0.790 |
| Potassium chloride | 0.368 |
| Disodium EDTA | 0.250 |
| Trimethoprim | 0.125 |
| Sodium bisulfite | 0.050 |
| Benzyl alcohol | 2.000 |
| Propylene glycol | 1.000 |
| Purified water USP, q.s. | 100 |

By way of example of a novel wetting composition, the following were formulated (amounts in % w/w):

| | |
|---|---|
| Sodium bicarbonate | 0.100 |
| Sodium phosphate (tribasic) | 0.030 |
| Sodium biphosphate | 0.030 |
| Sodium chloride | 0.790 |
| Potassium chloride | 0.368 |
| Disodium EDTA | 0.100 |
| Hydroxyethylcellulose 250H | 0.400 |
| Polyvinyl alcohol | 1.000 |
| Polyvinylpyrrolidone (Plasdone C) | 0.500 |
| Trimethoprim | 0.025 |
| Sodium Bisulfite | 0.050 |
| Benzyl Alcohol | 0.200 |
| Propylene Glycol | 0.500 |
| Purified water USP, q.s. | 100 |

CLAIMS

1. A sterile contact lens cleaning composition which comprises trimethoprim and a cleaning component suitable for cleaning contact lenses.

2. A sterile contact lens wetting composition which comprises trimethoprim, benzyl alcohol, and a wetting agent.

3. A sterile contact lens disinfectant composition which comprises trimethoprim and benzyl alcohol.

4. A composition according to claim 1, which comprises from 0.5 to 2.0% w/w trimethoprim.

5. A composition according to claim 1, which comprises from 0.075 to 0.3% w/w trimethoprim.

6. A composition according to any preceding claim, which comprises from 0.005 to 8.0% w/w of an alkylaryl polyether alcohol and from 0.5 to 8.0% w/w of an amphoteric surface-active agent.

7. A composition according to claim 6, wherein the amphoteric surface-active agent is 2-cocoyl-2-imidazolinium lauryl sulfate-1-carboxymethyloxyethyl-1-carboxymethyl disodium and the alkylaryl polyether alcohol is an isooctylphenoxypolyethoxyethanol.

8. A composition according to any preceding claim, which comprises a polyoxypropylene-polyoxyethylene block copolymer having a molecular weight of from 1,100 to 14,000 and a water solubility in excess of 100 g/l.

9. A composition according to any preceding claim, which has a tonicity of from 1.0 to 2.0.

10. A method for cleaning a contact lens, which comprises using a cleaning composition according to any preceding claim.

11. A composition according to claim 2, which comprises from 0.01 to 2.0% by weight trimethoprim.

12. A composition according to claim 2 or claim 11, which comprises from 0.1 to 5.0% w/w benzyl alcohol.

13. A composition according to any of claims 2, 11 and 12, which comprises 0.025 to 0.5% w/w ethylenediaminetetraacetic acid or a water-soluble salt thereof.

14. A composition according to any of claims 2, 11, 12 and 13, which comprises a water-soluble cellulosic polymer viscosity building agent selected from hydroxyethylcellulose, methylcellulose, carboxymethylcellulose and natural gums.

15. A composition according to claim 11, which comprises from 0.1 to 5.0% w/w benzyl alcohol, from 0.025 to 0.5% w/w ethylenediaminetetraacetic acid or a water-soluble salt thereof, from 0.05 to 0.80% w/w hydroxyethylcellulose, from 0.5 to 2.5% w/w polyvinyl alcohol, and from 0.5 to 2.0% w/w polyvinylpyrrolidone.

16. A composition according to claim 15, which comprises, by weight of the total composition, about 0.025% trimethoprim, about 0.2% benzyl alcohol, about 0.40% hydroxyethylcellulose, about 1.0% polyvinyl alcohol, about 0.5% polyvinylpyrrolidone, about 0.1% ethylenediaminetetraacetic acid disodium salt, about 0.10% sodium bicarbonate, about 0.030% sodium phosphate, about 0.050% sodium bisulfite, about 0.790% sodium chloride, about 0.368% potassium chloride, about 0.030% sodium biphosphate and about 0.50% propylene glycol.

17. A composition according to any of claims 2 and 11 to 16, which comprises a buffer for maintaining the pH of the composition in the range of from 7.0 to 8.0.

18. A method of wetting a silicone copolymer contact lens having a surface charge, characterized by neutralizing the surface charge, and contacting the lens with a wetting composition comprising a wetting component selected from polyvinyl alcohol, polyvinylpyrrolidone and cellulosic polymers.

19. A method according to claim 18, wherein the wetting composition is a composition according to any of claims 2 and 11 to 17.

20. A method according to claim 18 or claim 19, wherein the surface charge is neutralized by contacting the lens with a non-ionic detergent, and rinsing the detergent from the lens prior to contact with said wetting composition.

21. A composition according to claim 3, which comprises from 0.01 to 4.0% w/w trimethoprim and from 0.10 to 5.0% w/w benzyl alcohol.

22. A composition according to claim 21, which comprises about 0.125% w/w trimethoprim and about 2.0% w/w benzyl alcohol.

23. A composition according to any of claims 3, 21 and 22, which comprises ethylenediaminetetraacetic acid or a water-soluble salt thereof.

24. A composition according to any of claims 3, 21, 22 and 23, which comprises a buffer.

25. A composition according to claim 22, which comprises about 0.25% w/w ethylenediaminetetraacetic acid disodium salt, about 0.10% w/w sodium bicarbonate, about 0.03% w/w sodium phosphate, about 0.03% w/w sodium bisphosphate, about 0.79% w/w sodium chloride, about 0.368% w/w potassium chloride, about 0.05% w/w sodium bisulfite and about 1.0% w/w propylene glycol.

26. A method for treating a contact lens, to kill bacteria associated therewith, which comprises contacting the lens with a composition according to any of claims 3 and 21 to 25.

27. A method according to claim 26, wherein the lens is immersed in the composition for about 6 hours.